Europäisches Patentamt

European Patent Office

Office européen des brevets

(11) Veröffentlichungsnummer: **0 111 918**
A2

# (12) EUROPÄISCHE PATENTANMELDUNG

(21) Anmeldenummer: 83112770.9

(22) Anmeldetag: 19.12.83

(51) Int. Cl.³: **A 61 M 1/03**

(30) Priorität: 21.12.82 DE 3247149

(43) Veröffentlichungstag der Anmeldung:
27.06.84 Patentblatt 84/26

(84) Benannte Vertragsstaaten:
AT BE CH DE FR GB IT LI LU NL SE

(71) Anmelder: Krämer, Norbert
Röntgenstrasse 68
D-6100 Darmstadt 12(DE)

(72) Erfinder: Krämer, Norbert
Röntgenstrasse 68
D-6100 Darmstadt 12(DE)

(74) Vertreter: Mierswa, Klaus, Dipl.-Ing.
Tullastrasse 19
D-6800 Mannheim(DE)

(54) Vorrichtung zur Überwachung des Blutstandes in einem Oxygenator.

(57) Die Erfindung betrifft eine Vorrichtung zur Überwachung des Blutstandes in einem Oxygenator (48) mit flexiblen Versorgungsleitungen (21, 22, 23, 24, 25) zur Verhinderung des Eindringens von Sauerstoff in die Blutbahn des Patienten bei Rückleitung des mit Sauerstoff angereicherten Blutes aus dem Oxygenator, der mittels einer Halterung (42) gehaltert ist. Diese Halterung (42) weist eine Wägeeinrichtung (45) auf, mittels der der Oxygenator zur Ermittlung seines Gewichtes in Wägestellung verbunden ist. Die Versorgungsleitungen sind mittels einer Zugentlastung (5) ortsfest klemmend gehaltert, so daß das Gewicht der Teilstücke (36, 37, 38, 39, 40) der Versorgungsleitungen zwischen den Klemmstellen (26, 27, 28, 29, 30, 31) der Zugentlastung und dem Oxygenator konstant ist. Diese Teilstücke gehen gewichtsmäßig als Konstante in die Wägung ein und verfälschen somit nicht Wägeergebnis des Blutes innerhalb des Oxygenators.

Croydon Printing Company Ltd.

./...

Fig. 4

Die Erfindung geht aus von einer Vorrichtung gemäß dem Oberbegriff des Patentanspruchs 1.

Derartige Oxygenatoren werden bei Herz- Lungenoperationen eingesetzt, um das aus dem Körper des Patienten abgeleitete Blut außerhalb des Körpers, und zwar im Oxygenator mit Sauerstoff zu versorgen. Im Oxygenator findet eine Sauerstoffanreicherung des Blutes statt, wonach das Blut aus dem Oxygenator wieder dem Körper des Patienten zugeführt wird.

Während dieses Vorganges muß der Blutstand innerhalb des Oxygenators genau überwacht werden, damit beim Rückleiten des Blutes in den Körper des Patienten keine Luft oder kein Sauerstoff in die Blutbahn gelangt.

Es ist dabei bekannt, eine derartige Überwachung mittels optischen Signalen durchzuführen, wobei jedoch der Oxygenator aufgrund des darin befindlichen Blutes praktisch licht-undurchlässig ist und auch beim Rückleiten des Blutes ein Blutfilm auf der Innenwandung des Oxygenators haften bleibt. Deshalb wird heute in aller Regel die Rückleitung des Blutes aus dem Oxygenator von der Bedienungsperson der Herz-Lungen-Maschine direkt überwacht, was jedoch ebenfalls zu gefährlichen Fehlern führen kann, weil sich, wie gesagt, auf der Innenwandung des Oxygenators ein Blutfilm niederschlägt und deshalb der Blutstand innerhalb des Oxygenators nicht mit genügender Genauigkeit abgelesen werden kann.

Der Erfindung liegt deshalb die Aufgabe zugrunde, eine Vorrichtung der eingangs genannten Gattung zu schaffen, mit der der Blutstand innerhalb eines Oxygenators selbsttätig und vor allen Dingen mit hoher Genauigkeit überwacht werden kann.

Die Lösung dieser Aufgabe besteht im kennzeichnenden Teil des Patentanspruchs 1.

Weitere vorteilhafte Ausgestaltungen der Erfindung sind in den Unteransprüchen gekennzeichnet.

Die erfindungsgemäße Vorrichtung besitzt den hervorstechenden Vorteil, daß mit dieser der Blutstand innerhalb des Oxygenators über das Gewicht des Blutes innerhalb des Oxygenators bestimmt werden kann. Dabei sind aufgrund der erfindungsgemäß vorgesehenen Zugentlastung Fehlmessungen aufgrund der beweglichen Versorgungsleitungen des Oxygenators ausgeschlossen, weil die Teilstücke der Versorgungsleitungen zwischen dem Oxygenator und der Zugentlastung bzw. der Klemmleiste in das Gesamtgewicht des Oxygenators eingehen, ohne daß sich das Gewicht dieser Teilstücke der Versorgungsleitungen verändern kann. Dadurch kann das Gewicht des Blutes und somit die Blutmenge innerhalb des Oxygenators genau bestimmt werden.

In vorteilhafter Weise kann das Gewicht des Blutes innerhalb des Oxygenators laufend elektronisch gemessen werden, wobei die dergestalt gelieferten Meßimpulse in einer elektrischen Auswertschaltung ausgewertet werden. Innerhalb dieser Auswertschaltung können beispielsweise ein oder mehrere Werte vorgegeben werden, wobei bei Erreichen dieser Gewichtswerte ein Signal ausgelöst wird oder beispielsweise die Rückleitung des Blutes beendet wird. In vorteilhafter Weise ist es dadurch nicht mehr möglich, daß Sauerstoff aus dem Oxygenator in die Blutbahn des Patienten gelangt, wobei dadurch auch Ablesefehler der Bedienungsperson ausgeschlossen sind.

Drei Beispiele der Erfindung sind in der Zeichnung dargestellt und anschließend beschrieben. Dabei zeigen:

Figur 1 eine Ansicht einer erfindungsgemäßen Vorrichtung mittels eines C-förmigen Bügels, zwischen dessen C-Enden der Oxygenator in Wägestellung gehaltert ist, mit Dehnungsmeßdose und elektronischer Auswertung

Figur 2 eine Detaillzeichnung des Bügels und der Zugentlastung gemäß Figur 1

Figur 3 eine Draufsicht auf die Detaillzeichnung gemäß Figur 2

Figur 4 ein weiteres Beispiel einer erfindungsgemäßen Vorrichtung, die einen ungefähr waagrechten Arm besitzt, auf der eine Dehnungsmeßdose angeordnet ist, auf der wiederum der Oxygenator frei aufsitzt und

Figur 5 ein weiteres Beispiel der Erfindung mit einem Bügel, an dessen freiem Ende eine Dehnungsmeßdose angeordnet ist, an der der Oxygenator freihängend in Wägestellung aufgehängt ist.

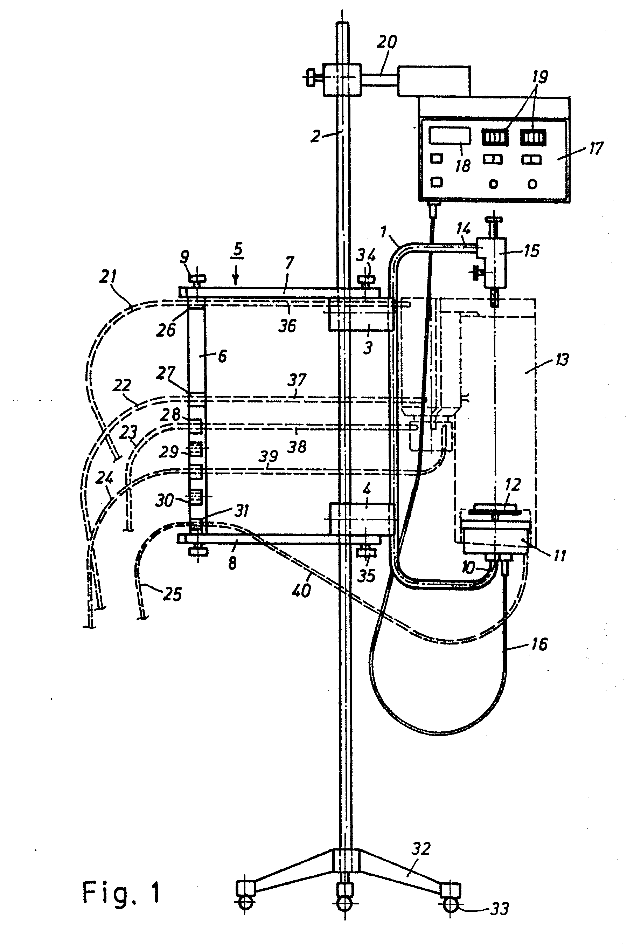

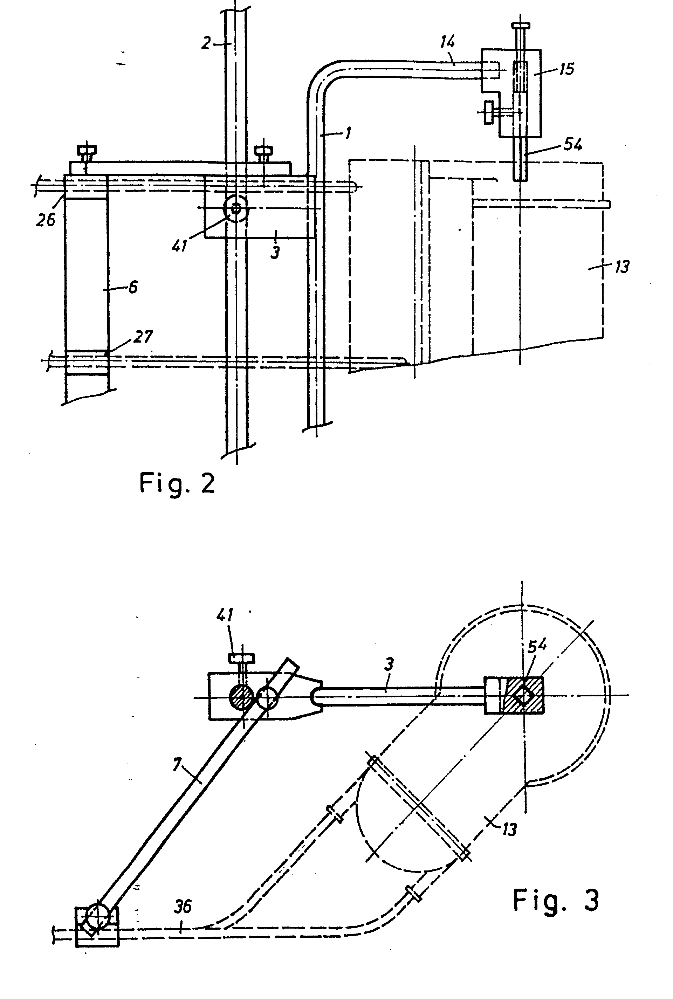

Das in den Figuren 1, 2 und 3 gezeigte Beispiel besteht aus einem fahrbaren Ständer 2, an dem verstellbar eine Zugentlastung 5 angeordnet ist, die einen Bügel 1 haltert. Der Bügel 1 weist zwei senkrecht übereinander angeordnete Arme 3, 4 auf, die Bohrungen aufweisen, durch die der Ständer 2 geführt ist. Mittels quer zu den Bohrungen angeordneter Klemmschrauben 41 (Figur 2) kann der Bügel 1 beliebig bezüglich seiner Höhe am Ständer 2 fixiert werden.

Das untere, zurück nach oben gebogene Ende 10 des Bügels 1 trägt eine Dehnungsmeßdose 11, innerhalb der ein Teller 12 gelagert ist, der auf die Meßzelle innerhalb der Dehnungsmeßdose drückt. Auf diesen Teller 12 und auf die Dehnungsmeßdose 11 ist nun ein Oxygenator 13 aufgesetzt, so daß derselbe auf dem Teller 12 aufsitzt und bezüglich der Dehnungsmeßdose 11 relativ beweglich ist. Am oberen Ende 14 des Bügels 1 ist eine verstellbare Klemme 15 mit einem nach unten ragenden Vierkantbolzen 54 (Fig. 2) angeordnet, wobei dieser Vierkantbolzen 54 in eine entsprechende Aussparung in der Oberseite des Oxygenators 13 hineinragt. Dabei wird der Oxygenator 13 durch die verstellbare Klemme 15 nicht geklemmt, sondern nur gehaltert, so daß der Oxygenator sich relativ zur Dehnungsmeßdose 11 bewegen kann. An der Dehnungsmeßdose 11 greift eine Meßleitung 16 an, die zu einer elektronischen Auswerteeinrichtung 17 führt, welche beispielsweise eine Anzeige 18 und zwei Voreinstellungen 19 aufweist. Die Auswerteeinrichtung 17 ist an einem Haltearm 20 mit Klemme gehaltert, wobei der Haltearm wiederum höhenverstellbar auf dem Ständer 2 befestigt ist.

An den Armen 3, 4, die zur Halterung des ungefähr C-förmigen Bügels 1 dienen, ist desweiteren eine Zugentlastung 5 angeordnet, so daß bei Verstellen des Bügels 1 mittels der Klemmschrauben 41 auch gleichzeitig die Zugentlastung 5 zwangsweise höhenverstellt wird. Somit bilden der Bügel 1, die Arme 3, 4 und die Zugentlastung 5 eine miteinander verbundene Einheit, was bei den übrigen Ausführungsbeispielen ebenfalls der Fall ist.

Die Zugentlastung 5 kann beispielsweise aus zwei senkrecht übereinander angeordneten Quertraversen 7, 8 bestehen, die an einem Ende mittels Klemmschrauben 34, 35 auf die Arme 3, 4 geschraubt sind. Mittels dieser Klemmschrauben 34, 35 können somit die Quertraversen 7, 8 in einer horizontalen Ebene geschwenkt werden. Die anderen Enden der Quertraversen 7, 8 sind mittels einer senkrecht angeordneten Klemmeinrichtung 6 miteinander verbunden, die vorzugsweise eine Klemmleiste ist. Diese Klemmleiste ist mittels Klemmschrauben 9 zwischen den Quertraversen 7, 8 beweglich gehaltert. Durch Lösen dieser Klemmschrauben 9 kann somit die Klemmleiste 6 senkrecht längs der Quertraversen 7, 8 in horizontaler Richtung verstellt werden. Die Klemmleiste 6 weist Klemmhalterungen 26, 27, 28, 29, 3o, 31 auf, mittels denen die Versorgungsleitungen 21, 22, 23, 24, 25 des Oxygenators 13 an die Klemmleiste 6 festgeklemmt werden, jedoch ohne die Versorgungsleitungen, die in der Regel flexibel sind, abzuklemmen. Die Klemmhalterungen 26, 27, 28, 29, 3o, 31 können Gummiklemmen oder Schellen sein, sie können bekannte Schnellverschlüsse aufweisen. Durch die Klemmhalterung der Versorgungsleitungen werden die Teilstücke 36, 37, 38, 39, 4o der Versorgungsleitungen zwischen dem Oxygenator 13 und der Klemmleiste 6 in ihrer Länge festfixiert, so daß diese Teilstücke gewichtsmäßig bei Befüllung der Leitungen konstant gehalten werden und somit keine Verfälschung der Gewichtsanzeige des Oxygenators durch Bewegung der Versorgungsleitung auftreten kann. Die Teilstücke der Versorgungsleitung zwischen dem Oxygenator 13 und der Klemmleiste 6 sind somit gewichtsmäßig konstant, gemessen wird nur das Gewicht des Blutes innerhalb des Oxygenators 13 nach Abzug der Leergewichtes desselben einschließlich dieser Teilstücke der Versorgungsleitungen. Damit können diese Teilstücke auch nicht gewichtsverfälschend in die laufenden Messungen eingehen.

Der Ständer 2 kann desweiteren einen fahrbaren Fuß 32 mit Rollen 33 aufweisen, so daß die Vorrichtung mitsamt dem Oxygenator beliebig verfahren werden kann.

Zur Arbeitsweise der erfindungsgemäßen Vorrichtung ist noch auszuführen, daß durch die elektronische Auswerteinrichtung eine Überwachung des Blutstandes des Blutes innerhalb des Oxygenators dergestalt stattfinden kann, daß beispielsweise zwei oder mehrere Alarmpunkte elektronisch gesetzt werden, die bei Erreichen des zugehörigen Gewichtes der verbleibenden Restmenge des Blutes einen Alarm geben oder beispielsweise die Rückleitung des Blutes unterbrechen. Deshalb kann der gesamte Vorgang der Rückleitung des mit Sauerstoff angereicherten Blutes selbsttätig ohne Überwachung durch eine Bedienungsperson durchgeführt werden.

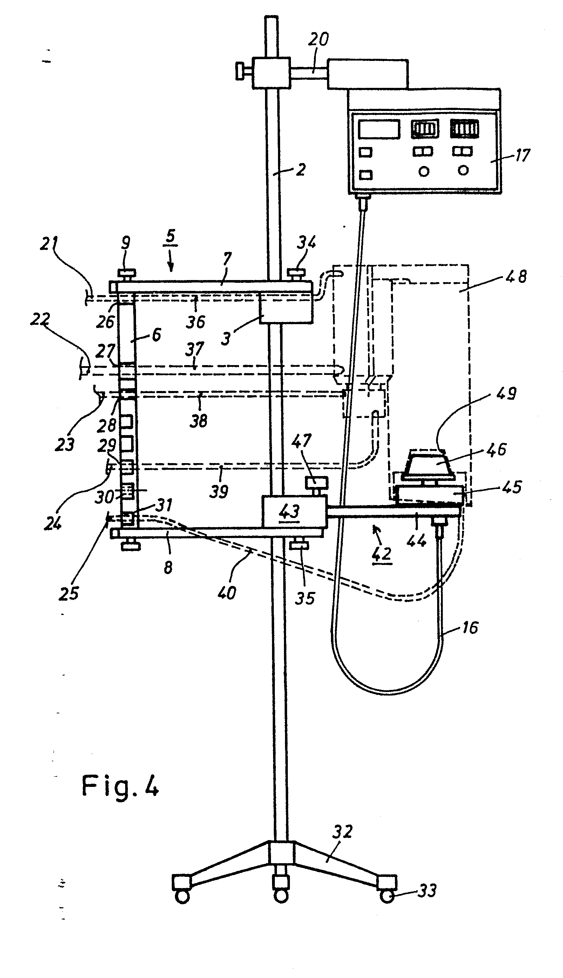

Fig. 4 zeigt ein weiteres Beispiel der erfindungsgemäßen Vorrichtung, bei der ein Oxygenator 48 auf eine Halterung 42 frei aufgesetzt ist, die aus einem Arm 44 besteht, der ungefähr waagrecht innerhalb eines Haltearmes 43 angeordnet ist, der in seiner Funktion dem Haltearm 4 der Figur 1 entspricht. Dabei ist der Arm 44 mittels einer Schraube 47 arretierbar. Am freien Ende des Armes 44 ist eine Dehnungsmeßdose 45 angeordnet, auf der eine kegelstumpfförmige Kappe 46 aufsitzt, die mit der Meßzelle der Dehnungsmeßdose 45 in Wirkverbindung steht. Der Oxygenator 48 besitzt an seinem unteren Ende, wie in Figur 4 gezeigt, eine entsprechend der Kappe 46 kegelstumpfförmig geformte Aussparung 49, mit der der Oxygenator 48 auf der Kappe 46 frei aufsitzt. Dabei ist der Oxygenator 48 gewichtsmäßig so ausbalanciert, daß der Schwerpunkt desselben bei Befüllung ungefähr oberhalb der Mitte der Kappe 46 bzw. der Dehnungsmeßdose 45 angeordnet ist. Die übrigen Teile der Vorrichtung gemäß Figur 4 entsprechen denen der Figur 1, weshalb auf die dort gegebene Beschreibung verwiesen wird.

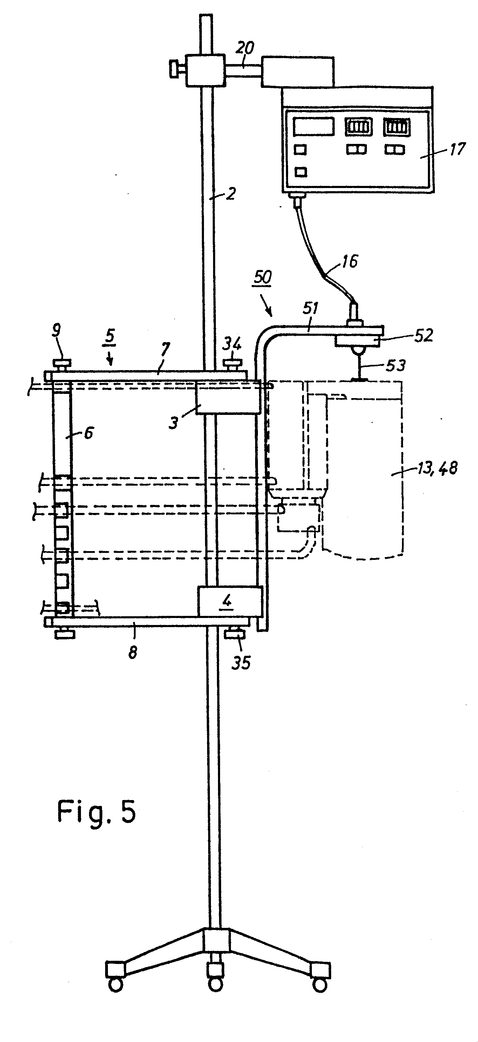

Ein weiteres Beispiel der Erfindung ist in Figur 5 gezeigt, die wiederum aus dem verfahrbaren Ständer 2 mit daran angeordneten, feststellbaren Armen 3, 4 und der Zugentlastung 5 besteht. Die Arme 3, 4 tragen hier eine Halterung 5o, die aus einem Bügel 51 mit waagrechter Abbiegung, also ungefähr umgekehr L-förmig, besteht. An der waagrechten Abbiegung des Bügels 51 ist beispielsweise eine elektrische Dehnungsmeßdose 52 entsprechend den Dehnungsmeßdosen 11 und 45 der Figuren 1

0111918

und 4 angeordnet. An der Dehnungsmeßdose 52 ist ein Draht 53
oder eine Stange befestigt, an der der Oxygenator 13, 48 frei
hängend aufgehängt ist.

Hier kann die Wiegeeinrichtung auch eine mechanische Einrichtung
sein, beispielsweise eine Zugfeder, die einen elektronischen
Analog-Digital-Wandler aufweist, der die analogen Meßwerte der
Zugfeder in elektronische Impulse umsetzt.

Gemäß den Ausführungsbeispielen der Figuren 1 und 4 ist auch in Fig.5
hier an den Armen 3, 4 der Halterung die Zugentlastung 5 angeordnet, die aus den Quertraversen 7, 8 und aus der Klemmleiste
6 besteht, die die beiden Quertraversen 7, 8 miteinander verbindet. Die übrige Ausgestaltung entspricht wiederum derjenigen
der Beispiele der Figuren 1 oder 4, weshalb auf die dortige
Beschreibung verwiesen wird.

Die drei genannten Beispiele unterscheiden sich dementsprechend
nur in der Ausgestaltung des Bügels bzw. des Armes und dadurch,
daß in den ersten beiden Ausführungsbeispielen der Oxygenator
auf die Wägeeinrichtung aufgesetzt und beim dritten Ausführungsbeispiel der Oxygenator an der Wägeeinrichtung aufgehängt ist.
Dabei kann die Aufhängung gemäß dem Ausführungsbeispiel der
Figur 5 dergestalt sein, daß bei Verwendung einer Dehnungsmeßdose diese ebenfalls auf Druck belastet wird.

Liste der Bezugszeichen

| | | |
|---|---|---|
| 1 | | Bügel |
| 2 | | Ständer |
| 3, 4 | | Arme |
| 5 | | Zugentlastung |
| 6 | | Klemmleiste |
| 7, 8 | | Quertraversen |
| 9 | | Klemmschraube |
| 1o | | unteres, rückgebogendes Ende |
| | | des Bügels 1 |
| 11 | | Dehnungsmeßdose |
| 12 | | Teller |
| 13 | | Oxygenator |
| 14 | | oberes Ende des Bügels 1 |
| 15 | | verstellbare Klemme |
| 16 | | Meßleitung |
| 17 | | elektronische Auswerteinrichtung |
| 18 | | Anzeige |
| 19 | | Voreinstellung |
| 2o | | Haltearm mit Klemme |
| 21, 22, 23, 24, 25 | | Zuführleitung des Oxygenators |
| 26, 27, 28, 29, 3o, 31 | | Klemmhalterungen der Klemmleiste |
| | | 6 für die Zuführleitungen |
| 32 | | Fuß |
| 33 | | Rollen |
| 34, 35 | | Klemmschrauben |
| 36, 37, 38, 39, 4o | | zugentlastete Endstücke der |
| | | Zuführleitung |
| 41 | | Klemmschraube |
| 42 | | Halterung |
| 43 | | Haltearm |
| 44 | | Waagrechter Arm |
| 45 | | Dehnungsmeßdose |
| 46 | | Kegelstumpfförmige Kappe |

| | |
|---|---|
| 47 | Klemmschraube |
| 48 | Oxygenator |
| 49 | Kegelstumpfförmige Aussparung |
| 5o | Halterung |
| 51 | Umgekehrt L-förmiger Bügel |
| 52 | Dehnungsmeßdose oder Wägeeinrichtung |
| 53 | Draht oder Stange |
| 54 | Vierkantbolzen |

Patentansprüche

1. Vorrichtung zur Überwachung des Blutstandes in einem Oxygenator mit flexiblen Versorgungsleitungen zur Verhinderung des Eindringens von Luft in die Blutbahn des Patienten bei Rückleitung des sauerstoffangereicherten Blutes aus dem Oxygenator zum Patienten, bestehend aus einer Halterung für den Oxygenator, dadurch gekennzeichnet, daß die Halterung (1, 42, 5o) eine Wägeeinrichtung (11, 12; 45, 46; 52, 53) aufweist, mittels der der Oxygenator (13, 48) zur Ermittlung seines Gewichts in Wägestellung verbunden ist und die Versorgungsleitungen (21, 22, 23, 24, 25) mittels einer Zugentlastung (5) ortsfest klemmend gehalten sind zur Konstanthaltung des Gewichts von Teilstücken (36, 37, 38, 39, 4o) dieser Versorgungsleitungen zwischen Klemmstellen (26, 27, 28, 29, 3o) der Zugentlastung (5) und dem Oxygenator (13, 48).

2. Vorrichtung nach Anspruch 1, gekennzeichnet durch folgende Merkmale:

a) die Halterung ist ein Bügel (1, 51) oder Arm (42), an dem der Oxygenator (13, 48) beweglich in Wäge- position gehalten ist,

b) der Bügel (1, 51) oder Arm (44) weist eine Dehnungsmeßdose (11, 52, 45) als Wägevorrichtung auf, an der der Oxygenator (13, 48) in Wägestellung gehaltert ist,

c) die Zugentlastung (5) zur Halterung der Versorgungsleitungen (21, 22, 23, 24, 25) des Oxygenators (13, 48) weist eine Klemmeinrichtung (6), insbesondere Klemmleiste, auf, in der die Versorgungsleitungen klemmend gehaltert sind,

d) die Vorrichtung besitzt eine elektronische Auswertschaltung (17) zur Auswertung der Meßimpulse der
Dehnungsmeßdose (11, 45, 52).

3. Vorrichtung nach Anspruch 2,
dadurch gekennzeichnet,
daß die Halterung ein C-förmiger Bügel (1) ist, zwischen
dessen Enden (1o, 14) der Oxygenator (13) beweglich in
Wägestellung gehaltert ist, wobei die Dehnungsmeßdose
(11) auf das untere Ende (1o) des Bügels aufgesetzt ist
und der Oxygenator auf der Dehnungsmeßdose aufsitzt.

4. Vorrichtung nach Anspruch 2,
dadurch gekennzeichnet,
daß der Arm (44) waagrecht angeordnet ist und an seinem
freien Ende die Dehnungsmeßdose (45) trägt, auf der
eine kegelstumpfförmige Kappe (46) angeordnet ist, auf
der der Oxygenator (48) mit einer entsprechend geformten
kegelstumpfförmigen Aussparung (49) frei aufsitzt.

5. Vorrichtung nach Anspruch 1,
dadurch gekennzeichnet,
daß der Bügel (51) oder Arm waagrecht angeordnet ist, wobei am freien Ende des Bügels oder Arms die Dehnungsmeßdose (52) angeordnet ist, an der der Oxygenator (13,
48) mittels einer Stange oder eines Drahtes (52) frei
hängend aufgehängt ist.

6. Vorrichtung nach Anspruch 2,
dadurch gekennzeichnet,
daß die Zugentlastung (5) mit dem Bügel (1, 51) oder Arm
(44) verbunden ist und beide Teile gemeinsam höhenverstellbar an einem fahrbaren Ständer (2) angeordnet sind.

7. Vorrichtung nach Anspruch 6,
dadurch gekennzeichnet,
daß die Zugentlastung (5) aus zwei parallel übereinander angeordneten Quertraversen (7, 8) besteht, deren ein Ende am Bügel (1, 51) oder Arm (44) befestigt sind und deren anderes Ende durch die Klemmeinrichtung (6) verbunden sind.

8. Vorrichtung nach Anspruch 7,
dadurch gekennzeichnet,
daß die Klemmleiste (6) in waagrechter Richtung längs der Quertraversen (7, 8) verstellbar ist.

9. Vorrichtung nach Anspruch 1,
dadurch gekennzeichnet,
daß die Wägevorrichtung eines Zugfeder (43) mit mechanischer Anzeige ist, die am oberen Ende der Halterung des Oxygenators angeordnet ist und an der derselbe aufgehängt ist.

Fig. 1

Fig. 2

Fig. 3

Fig. 4

0111918

Fig. 5